# EUROPEAN PATENT APPLICATION

(11) **EP 3 970 652 A1**
(43) Date of publication of application: **23.03.2022**
(21) Application number: 19928814.3
(22) Date of filing: 13.06.2019
(51) Int. Cl.: A61B 90/00

(54) **TRACKING DEVICE AND LOCALIZATION SYSTEM**

(30) Priority: 15.05.2019 CN 201910407904
(71) Applicant: Tinavi Medical Technologies Co., Ltd., Beijing 100192 (CN)
(72) Inventor: DENG, Mingming, Beijing 100192 (CN); ZHAO, Yongqiang, Beijing 100192 (CN); LI, Yinyan, Beijing 100192 (CN)
(74) Representative: Kurig, Thomas
(86) International application number: PCT/CN2019/091026
(87) International publication number: WO 2020/228087

(57) **Abstract**

The invention relates to a tracing device and a positioning system. The tracing device is used for indicating the spatial position of a mechanical arm of a surgical robot. The tracing device includes: a base, wherein the base has two opposite ends and an outer circumferential surface located between the two ends, one end is connected to an operation end of the mechanical arm, and a surgical instrument is installed at the other end; a positioning assembly, which is connected to the base and used for indicating the spatial position of the base; wherein the positioning assembly includes at least three tracing elements which are arranged on the outer circumferential surface in a non-collinear manner; wherein there is a plurality of positioning assemblies, and the plurality of positioning assemblies are distributed in a circumferential direction of the base; and a normal angle between any two of the tracing elements of each positioning assembly is smaller than or equal to 20°. According to the tracing device and the positioning system, the tracing device can be more easily recognized by a position finder during the rotation process of the mechanical arm.

## Description

This application claims priority to Chinese Invention Patent Application No. 201910407904.9 entitled "Tracing Device and Positioning System", and filed on May 15, 2019, which is incorporated by reference herein in its entirety.

### TECHNICAL FIELD

The invention relates to the technical field of positioning of medical instruments, and in particular to a tracing device and a positioning system.

### BACKGROUND

During surgical navigation, a navigation system acquires the position of the operation end of a mechanical arm in a space coordinate system referring to an optical position finder by means of a tracing device arranged at the operation end of the mechanical arm, so as to figure out the position of a surgical instrument fixed to the operation end of the mechanical arm to plan out the surgery path of the mechanical arm.

Due to the unreasonable configuration positions of tracing elements in an existing mechanical arm tracing device, an optical position finder may fail to recognize the position of the tracing device when the normal angle between the tracing elements of the tracing device and the optical position finder changes along with the rotation of the operation end of the mechanical arm, and consequentially, the navigation system loses the spatial position of the mechanical arm.

### BRIEF SUMMARY

The invention provides a tracing device and a positioning system, wherein the tracing device can be more easily recognized by an optical position finder during the rotation of a mechanical arm.

The invention provides a tracing device. The tracing device is used for indicating the spatial position of a mechanical arm of a surgical robot. The tracing device includes a base, which has two opposite ends and an outer circumferential surface located between the two ends, one end is connected to an operation end of the mechanical arm, and a surgical instrument is installed at the other end; a positioning assembly, which is connected to the base and are used for indicating the spatial position of the base, wherein the positioning assembly includes at least three tracing elements which are arranged on the outer circumferential surface of the base in a non-collinear manner; wherein there is a plurality of positioning assemblies, and the plurality of positioning assemblies are distributed in a circumferential direction of the base, and a normal angle between any two of the tracing elements of each positioning assembly is smaller than or equal to 20°.

In one embodiment of the invention, the plurality of positioning assemblies are arranged in the full circumferential direction of the base; and in every two adjacent positioning assemblies, a normal angle between any one of the tracing elements of one positioning assembly and any one of the tracing elements of the other positioning assembly is smaller than or equal to 90°.

In one embodiment of the invention, normal directions of the tracing elements of each positioning assembly are consistent.

In one embodiment of the invention, five positioning assemblies are arranged in the full circumferential direction of the base; in every two adjacent positioning assemblies, teh normal angle between any one of the tracing elements of one positioning assembly and any one of the tracing elements of the other positioning assembly is 72°; and/or, the angle between a vertical line from any point on an outer side of the base to the outer circumferential surface and the normal direction of each tracing element of at least one positioning assembly is smaller than or equal to 36°.

In one embodiment of the invention, every two adjacent positioning assemblies are spaced from each other; or, every two adjacent positioning assemblies are staggered with each other.

In one embodiment of the invention, the at least three tracing elements of the positioning assembly are configured in a preset distribution pattern, and the distribution patterns of the tracing elements of any two positioning assemblies are different, so that an optical position finder can distinguish the positioning assemblies according to the distribution patterns.

In one embodiment of the invention, at least one end of the base is provided with a positioning assembly, which includes a positioning hole and/or positioning protrusion located at the end.

In one embodiment of the invention, the base is of a columnar structure.

In one embodiment of the invention, the base is provided with a plurality of installation parts, which include installation planes and/or installation holes, and each installation part is provided with at least one of the tracing elements.

In one embodiment of the invention, the base is further provided with recessed surfaces in one-to-one correspondence with the installation parts.

In one embodiment of the invention, the base includes a first column and a second column in the axial direction of the base, the first column and the second column are arranged coaxially, the outer diameter of the first column is greater than that of the second column, and a connecting groove is formed in one end, away from the second columnar, of the first column and extends in the axial direction.

In one embodiment of the invention, the length of a line segment formed by connecting distribution points of any two tracing elements of each positioning assembly has is greater than 40mm; and/or the absolute value of a length difference of any two of multiple line segments formed by connecting of any two tracing elements of each positioning assembly is greater than 3.5mm.

In one embodiment of the invention, the tracing device further includes a signal receiver, which is connected to the base and used for receiving a starting signal, and the tracing elements transmit spatial positional information according to the starting signal.

In one embodiment of the invention, the tracing elements are infrared emitters or reflection balls.

The invention further provides a positioning system. The positioning system includes an host computer, an optical position finder, a surgical robot, a calibration assembly, and the tracing device mentioned above, wherein the tracing device is installed at an operation end of a mechanical arm of the surgical robot, and the calibration assembly is in a preset positional relationship with the tracing elements on the tracing device in the image registration process; the host computer, through an acquired wound image including marker information of the calibration assembly and the spatial positional information of the tracing elements of the tracing device acquired by the optical position finder, converts a wound image coordinate system into a coordinate system referring to the tracing elements or into a coordinate system referring to the optical position finder, so as to complete image registration; and the host computer controls the mechanical arm of the surgical robot to reach a target spatial position corresponding to a planned path according to the panned path on the wound image.

In one embodiment of the invention, the positioning system further includes a patient tracer, the optical position finder acquires spatial positional information of the patient tracer at a certain frequency, and the host computer corrects the path of the surgical robot according to the acquired spatial positional information of the patient tracer.

According to the tracing device and the positioning system of the invention, the tracing device can be recognized by the optical position finder within a wider range through the plurality of positioning assemblies arranged in the circumferential direction of the base. Meanwhile, the normal angle between any two of the tracing elements of each positioning assembly is limited to be smaller than or equal to 20°, so that the tracing device can be more easily recognized by the optical position finder during the rotation process of the mechanical arm, the situation that the optical position finder loses the position of the tracing device when the mechanical arm rotates is avoided, and the positioning precision is improved.

### BRIEF DESCRIPTION OF THE SEVERAL VIEWS OF THE DRAWINGS

The characteristics, advantages and technical effects of illustrative embodiments of the invention are described below with reference to the accompanying drawings.
FIG 1 is an axonometric drawing of a tracing device according to an embodiment of the invention;
FIG 2 is a front view of the tracing device according to the embodiment of the invention;
FIG. 3 is a sectional view along M-M in FIG. 2;
FIG. 4 is a sectional view along N-N in FIG. 2;

### Reference Signs:

10, base; 11, first column; 111, connecting groove; 12, second column; 13, installation plane; 14, recessed surface; 101, end; 102, outer circumferential surface;
20, positioning assembly; 21, tracing element;
30, positioning mechanism;
40, signal receiver;
X-circumferential direction; Y-axial direction

In the figures, identical components are presented by identical reference signs. The drawings are not draw to actual scale.

### DETAILED DESCRIPTION OF EMBODIMENTS

The features and illustrative embodiments of the invention are detailed below. For a comprehensive understanding to the invention, many details are given in the following description. However, it is obvious for those skilled in the art to implement the invention without certain ones of these specific details. The descriptions to embodiment are used for a better understanding of the invention by showing embodiments of this invention. At least part of well-known structures and techniques are not shown in the accompanying drawings and the following description, so as to avoid obscuring the invention. In addition, for the sake of a clear illustration, the size of a part of structures is exaggerated. Moreover, the features, structures, and characteristics in the following description can be combined in one or more embodiments in any appropriate way.

All directional terms involved in the following description refer to directions shown in the drawings and are not intended to limit the specific structure of the tracing device and the positioning system of this invention. What needs to be pointed out is that unless otherwise explicitly specified and qualified, the terms "install" and "connect" should be generally understood. For instance, the terms "install" and "connect" may refer to "fixed connection", "detachable connection", "integral connection", "direct connection", or "indirect connection". Those ordinarily skilled in the art can appreciate the specific meanings of these terms in the invention according to specific situations.

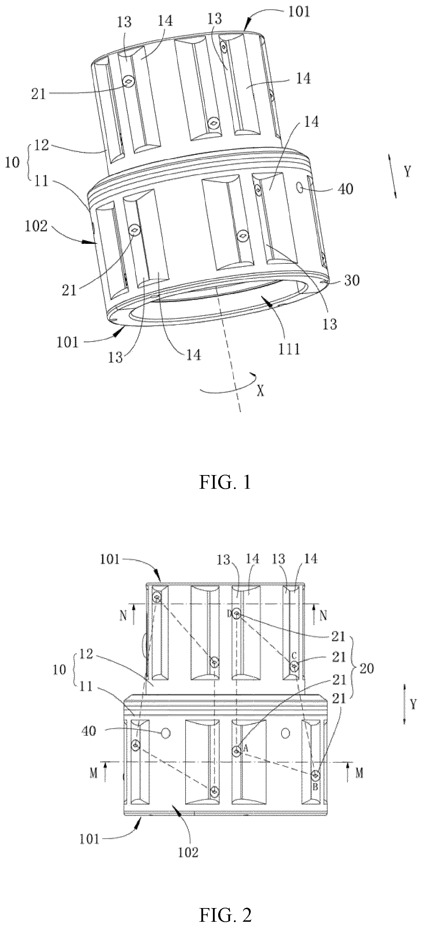

For a better understanding of the invention, the tracing device and positioning system of the invention are detailed below with reference to FIGs. 1-4.

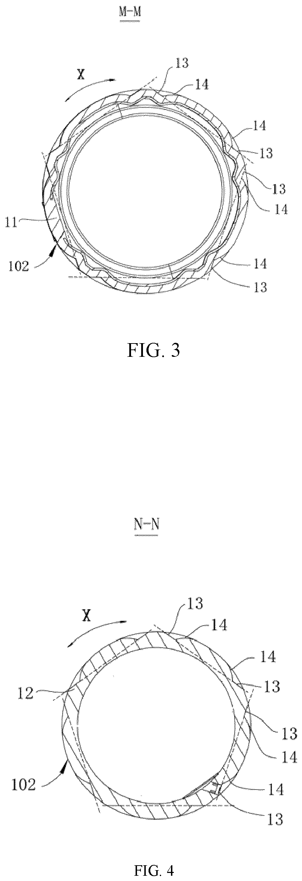

Please refer to FIGs. 1-4, wherein FIG. 1 is an axonometric drawing of the tracing device according to an embodiment of the invention, FIG. 2 is a front view of the tracing device according to an embodiment of the invention, FIG. 3 is a sectional view along M-M in FIG. 2, and FIG. 4 is a sectional view along N-N in FIG. 2.

The tracing device in this embodiment of the invention is used for indicating the spatial position of a mechanical arm of a surgical robot, and includes a base 10 and a positioning assembly 20. The base 10 has two opposite ends 101 and an outer circumferential surface 102 located between the two ends 101, wherein one end 101 is connected to an operation end of the mechanical arm, and a surgical instrument is installed at the other end 101. The surgical instrument mentioned above and below in this embodiment of the invention can be a guide used for installing a spicule sleeve. The positioning assembly 20 is arranged on the base 10 and is used for indicating the spatial position of the base 10. Each positioning assembly 20 includes at least three tracing elements 21 which are arranged on the outer circumferential surface 102 of the base 10 in a non-collinear manner. There is a plurality of assemblies 20, and the plurality of positioning assemblies 20 are distributed in a circumferential direction X of the base 10. The normal angle between any two of the tracing elements 21 of each positioning assembly 20 is smaller than or equal to 20°.

According to the tracing device and the positioning system in this embodiment of the invention, the tracing device can be recognized by an optical position finder in a wider range through the positioning assemblies 20 arranged in the circumferential direction X of the base 10. Meanwhile, the normal angle between any two of the tracing elements 21 of each positioning assembly 20 is limited to be smaller than or equal to 20°, so that the tracing device can be more easily recognized by the optical position finder during the rotation of the mechanical arm, the situation that the optical position finder loses the position of the tracing device when the mechanical arm rotates is avoided, and the positioning precision is improved.

Optionally, the normal angle between any two of the tracing elements 21 of each positioning assembly 20 is greater than or equal to 0° and smaller than or equal to 15°. Furthermore, the normal angle is greater than or equal to 0° and smaller than or equal to 10° such as 8°, 5°, 3° or 1°. By limiting the normal angle between the tracing elements 21 of each positioning assembly 20 within a certain range, the tracing device is further optimized to be better recognized by the optical position finder during rotation, and accordingly, the spatial position of the mechanical arm can be better determined.

Optionally, normal directions of the tracing elements 21 of each positioning assembly 20 are consistent, which means that the normal angle between any two tracing elements 21 of each positioning assembly 20 is 0°. Under this configuration, when the tracing device and the optical position finder are used in cooperation, as long as one tracing element 21 of each positioning assembly 20 is recognized by the optical position finder, three or more tracing elements 21 of the positioning assembly 20 can be recognized by the optical position finder without exception, then the position of the positioning assembly 20 is determined by the optical position finder. The positioning precision of the tracing device is improved through the tracing elements 21.

Optionally, the base 10 is a of columnar structure on the whole such as a hollow columnar structure, or partially-solid columnar structure. Optionally, the base 10 is of a cylindrical structure, and particularly, the base 10 is of a uniform-section cylindrical structure in an axial direction Y thereof, or a nonuniform-section cylindrical structure, for instance, a stepped columnar structure.

Referring to FIGs. 1-4 again, in certain optional embodiments, the base 10 includes a first column 11 and a second column 12 in the axial direction Y, wherein the first column 11 and the second column 12 are arranged coaxially, and the outer diameter of the first column 11 is greater than that of the second column 12; and a connecting groove 111 is formed in an end, away from the second column 12, of the first column 11 and extends in the axial direction Y. The base 10 of such structure can be arranged on the operation end of the mechanical arm through the connecting groove 111 so as to be connected to the mechanical arm more conveniently, and the connection stability is ensured. Meanwhile, the weight of the tracing device is reduced, the space occupation of the tracing device at the operation end of the mechanical arm is reduced, and accordingly, more surgical operation space is saved.

Optionally, the end 101 of the base 10 used to be connected to the mechanical arm is fixed to the operation end of the mechanical arm with a screw, and particularly, with a captive screw, so that the tracing device is unlikely to fall off.

Most traditional positioning assemblies are flat positioning assemblies. Large protrusions are formed when traditional flat positioning assemblies are fixed to the mechanical arm through brackets, and these protrusions need to be sterilized during surgery. Nowadays, the operation end of the mechanical arm needs to be wrapped with a sterile cover before surgery, and then the sterilized positioning assemblies and connecting devices used for installing the positioning assemblies are connected to the operation end of the mechanical arm, so that preoperative procedures are complex, and the preparation time is long.

According to the tracing device in this embodiment of the invention, the base 10 is columnar, and the tracing elements 21 are directly arranged on the side wall of the base 10. As such, different from traditional flat tracing devices, the tracing device in this embodiment can be arranged in the sterile cover, and thus, surgical operations are simplified, and the surgical time is shortened. In addition, the tracing device in this embodiment can be always fixed to the operation end of the mechanical arm through the connecting groove 111 formed in the corresponding end 101 of the tracing device and does not need to be disassembled, so that abrasion of the tracing device during assembly and disassembly is avoided, the dimensional precision of the tracing device is maintained, and the operating precision of the navigation system is improved.

Optionally, the end connected to the mechanical arm of the base 10 may be an insulator, so as to prevent currents in the tracing device from flowing towards operation instrument.

Optionally, in order to facilitate the installation of the tracing elements 21, the base 10 is provided with a plurality of installation parts allowing the tracing elements 21 to be installed thereon, so that the tracing elements 21 are protected while meeting the installation requirement of the tracing elements 21. Optionally, each installation part includes an installation plane 13 which extends from the outer circumferential surface 102 of the base 10 to the interior of the base 10 by a preset distance. The installation plane 13 can be an oblique plane which intersects with a plane across the central axis of the base. The installation plane 13 can also be configured in other ways as long as the installation requirement of the tracing elements 21 and the normal direction requirement of the tracing elements 21 are met after installation.

Optionally, the first column 11 and the second column 12 are provided with installation parts including installation planes 13, and at least one tracing element 21 is arranged on each installation plane 13. Optionally, in order to facilitate machining and to better meet the normal direction requirement of the tracing elements 21 of each positioning assemblies 20, the number of the installation parts on the first column 11 is equal to that of the installation parts on the second column 12, and the installation parts on the first column 11 are in one-to-one correspondence with the installation parts on the second column 12. In the case where the installation parts include installation planes 13, the number of the installation planes 13 on the first column 11 is equal to that of the installation planes 13 on the second column 12, and the installation planes 13 on the first column 11 are in one-to-one correspondence with the installation planes 13 on the second column 12. Furthermore, the installation planes 13 arranged on the first column 11 and the second column 12 in the one-to-one correspondence manner are in parallel, so as to facilitate machining and better meet the normal direction requirement of the tracing element 21.

Optionally, as shown in FIG. 3 and FIG. 4, every two installation planes 13 on the first column 11 form a set, and extensions of the two installation planes 13 in each set are located on the same plane; and/or, every two installation planes 13 on the second column 12 form a set, and extended surfaces of the two installation planes 13 in each set are in parallel. In this way, machining is facilitated, and the normal angle requirement of the tracing elements 21 is better met.

Optionally, the normal direction of at least one tracing element 21 is consistent with that of the connected installation plane 13, so that installation of the tracing elements 21 is further facilitated. Optionally, the normal direction of each tracing element 21 is consistent with that of the connected installation plane 13, so that the normal directions of the tracing elements 21 and the connection stability of the tracing elements 21 with the corresponding installation planes 13 are ensured, and the production efficiency of the tracing device is improved.

Clearly, installation parts including the installation planes 13 is merely one optical implementation, and it does not mean that the installation parts include the installation planes 13 only. In other embodiments, the installation parts include installation holes only, wherein at least one tracing element 21 is arranged in each installation hole. Optionally, the installation hole is a blind hole, the normal direction of the bottom surface forming the installation hole is consistent with that of the tracing element 21 in the installation hole, and in this way, the installation requirement and the normal angle requirement of the tracing elements 21 can also be met.

Clearly, in other embodiments, the installation parts include both installation planes 13 and installation holes. In this way, the installation requirement of the tracing elements 21 can be met, and the tracing elements 21 can adapt to different shapes of the base 10.

Optionally, in order to make sure that lights can reach the installation parts without being shielded by the outer surface of the base, the outer surface of the base needs to be designed to give way to lights from the tracing elements 21. Particularly, the base 10 of the tracing device in the above embodiments is further provided with recessed surfaces 14 which are in one-to-one correspondence with the installation parts, wherein the recessed surfaces 14 are planes, or curved surfaces such as cambered surfaces. In the case where an installation parts include an installation plane 13, the recessed surface 14 corresponding to the installation part is connected with one end of the installation plane 13 of the installation part, the other end of the recessed surface 14 extend away from the installation plane 13, so that a groove structure is formed by the installation plane 13 and the recessed surface 14, and the tracing element 21 can be recognized by the optical position finder more easily.

Optionally, the tracing element 21 is an infrared emitter which can meet the tracing requirement of the tracing device. Meanwhile, the two ends of the infrared emitter is of a planar columnar structure, so that the infrared emitter can be directly attached and connected to the corresponding installation plane 13 or into the installation hole to meet the normal direction requirement of the tracing elements 21. As such, the machining cost of the tracing device is further reduced, the space is saved, the positioning precision of the installation positions of the tracing elements 21 is ensured, and accordingly, the tracing device can better meet the requirement for the positioning precision of the mechanical arm.

Clearly, the tracing element 21 being an infrared emitter is merely one optical implementation. In other embodiments, the tracing element 21 can be a light-emitting ball, which can also meet the tracing requirement of the tracing device.

In the case where the tracing element 21 is an infrared emitter, the normal direction of the tracing element 21 is the direction perpendicular to light-emitting surface of the tracing element 21. In the case where the tracing element 21 is a light-emitting ball, the normal direction of the tracing elements 21 are the extension direction of the central lines of the light-emitting ball.

Optionally, according to the tracing device in this embodiment of this invention, the number of the tracing elements 21 of each positioning assembly 20 can be set as needed. For instance, the number of the tracing elements 21 of each positioning assembly 20 is three, four, five, or more, and can be set according to the requirement for the positioning precision and the arrangement ways of the tracing elements 21.

Optionally, the at least three tracing elements 21 of each positioning assembly 20 are arranged in a preset distribution pattern. Particularly, the preset distribution pattern of the tracing elements 21 is an asymmetric pattern. For instance, three tracing elements 21 respectively serve as the three vertices of a triangle having three unequal sides.

In this embodiment of the invention, the optical position finder determines three positioning points through three non-collinear tracing elements 21, and the three positioning points are connected to form a triangular template to determine a tracing surface, or to determine a coordinate system of the tracing surface. Because the positioning points are fixed to the side wall of the tracing device, the spatial positions of the three points can be compared with the triangular template of the preset distribution pattern by the optical position finder to figure out the spatial position coordinates of the tracing device in the coordinate system referring to on the optical position finder.

In certain embodiments, each positioning assembly 20 includes four or more tracing elements 21 arranged in a preset distribution pattern, wherein every three tracing elements 21 are not collinear; and when establishing the coordinate system of a tracing surface, the optical position finder acquires the spatial positional information of the tracing elements 21 and then establishes the coordinate system of the tracing surface according to the positional information of three points with the minimum error or deviation, so that the measurement error of the spatial position of the tracing device is reduced.

Optionally, at least three tracing elements 21 of each positioning assembly 20 are arranged in a preset distribution pattern, and the distribution patterns of the tracing elements 21 of any two positioning assemblies 20 are different, so that the optical position finder can distinguish the positioning assemblies 20 from one another according to the distribution patterns and can uniquely determine the spatial position coordinates of the tracing device according to the spatial position coordinates of the positioning points formed by the positioning assemblies 20.

In addition, in certain optional embodiments, according to the tracing device provided in the aforementioned embodiment, the length of a line segment formed by connection of the distribution points of any two tracing elements 21 of each positioning assembly 20 of the tracing device is greater than 40mm. Optionally, the length of the line segment formed by connection of the distribution points of any two tracing elements 21 on a same tracing surface 30 is 40mm-80mm, and optionally 40mm-60mm, such as 45mm, 50mm, and 55mm.

For a better understanding of the above dimension limitation, an example is given below. As shown in FIG. 2, point A, point B, point C and point D in FIG. 2 are the distribution positions of four tracing elements 21 of a same positioning assembly 20 and are sequentially connected to form a tracing surface. The length of any one of line segments AB, AC, AD, BC, BD and CD on the tracing surface should be greater than 40mm. In this way, overlapping of the distribution points, to be acquired by the optical position finders, of any two tracing elements 21 on each tracing surface 30 can be avoided, and accordingly, the positioning and adjustment precision of the mechanical arm is improved.

Optionally, according to the tracing device of the above embodiments, in the multiple line segments formed by connecting the distribution positions of every two tracing elements 21 of a same positioning assembly 20, the absolute value of a length difference between any two line segments is greater than 3.5mm.

For a better understanding of the above dimension limitation, an example is given below. As shown in FIG. 2, multiple line segments AB, AC, AD, BC, BD and CD are formed by connecting the distribution points of every two tracing elements 21 of a same positioning assembly 20, and the absolute values (|AB-AC|, |AB-AD|, |AB-BC|, |AB-BD|, |AB-CD|, |AC-AD|, |AC-BC|, |AC-BD|, |AC-CD|, |AD-BC|, |AD-BD|, |AD-CD|, |BC-BD|, |BC-CD|, and |BD-CD|) of length difference between any two line segments are greater than 3.5mm. In this way, the optical position finder can accurately distinguish the line segments formed by the distribution points of different tracing elements 21 on each tracing surface, and the posture judgment and adjustment precision of the mechanical arm is further improved.

Optionally, the number of the positioning assemblies 20 can be set according to actual needs, such as 3-8. The multiple positioning assemblies 20 are arranged in the full circumferential direction of the base 10, and particularly, the positioning assemblies 20 are arranged at different positions in the circumferential direction X of the base 10 and cover the base 10 in the full circumferential direction. When the base 10 rotates around the axial direction Y of its own, there is always one positioning assembly 20 that can be recognized at one point on the outer side of the base 10. For instance, when the tracing device and the optical position finder are used in cooperation, the axial direction Y of the base 10 is kept perpendicular to the normal direction of the optical position finer. When the base 10 rotates around the axis of its own, there is always one positioning assembly 20 that can be recognized by the optical position finder, so that it is ensured that the optical position finder can always acquire the spatial positional information of the tracing device through the base 10 even the base 10 is located at different positions, so as to realize real-time monitoring of the spatial position of the operation end of the mechanical arm.

For example, if the optical position finder is composed of two optical sensors, the normal direction of the optical position finder is the direction extending outward along the vertical line of the connection line between the two optical sensors within the plane determined by lens orientation of the two optical sensors of the optical position finder.

Optionally, in every two adjacent positioning assemblies 20 of the tracing device in the above embodiments, the normal angle between any one tracing element 21 of one positioning assembly 20 and any one tracing element 21 of the other positioning assembly 20 is smaller than or equal to 90°. In this way, when the tracing device and the optical position finder are used in cooperation, because the normal angle between any one tracing element 21 of one of two adjacent positioning assemblies 20 and any one tracing element 21 of the other positioning assembly 20 is not greater than 90°, the normal angle between the tracing elements 21 and the optical position finder is not greater than 45°. As such, the optical position finder can easily detect signals from the tracing elements 21, and normal and stable usage of the tracing device is ensured.

Optionally, in the tracing device according to the embodiments of this invention, five positioning assemblies 20 are arranged in the full circumferential direction of the base 10, wherein in every two adjacent positioning assemblies 20, the normal angle between the tracing elements 21 of one positioning assembly 20 and the tracing elements 21 of the other positioning assembly 20 is 72°, and the included angle between a vertical line from any point on the outer side of the base 10 to the outer circumferential surface 102 and the normal direction of the tracing elements 21 of at least one positioning assembly 20 is smaller than or equal to 36°. The recognizable angle of the tracing device can be further improved through the five positioning assemblies 20 arranged in the full circumferential direction X of the base 10, and the requirement for the installation precision of the positioning assemblies 20 is lowered. Under the comprehensive consideration of the installation and design precision and the recognizable angle of the tracing device, it is the optimal design to configure five positioning assemblies 20 in the full circumferential direction of the base.

During specific implementation, every two adjacent ones of the multiple positioning assemblies 20 of the tracing device are spaced from each other; of course , in other embodiments, every two adjacent ones of the multiple positioning assemblies 20 of the tracing device are staggered with each other, and both configurations can meet the tracing requirement of the tracing device. If the positioning assemblies are staggered with one another, the occupation of the outer surface of the base can be reduced, and the space occupied by the base 10 can be reduced.

Optionally, in the tracing device according to the embodiments of this invention, at least one end 101 of the base 10 is provided with a positioning mechanism 30 which includes a positioning hole and/or a positioning protrusion located at the end 101. The positioning mechanism 30 can fulfill factory calibration of the tracing device, particularly can achieve conversion of a three-coordinate system into a front-end coordinate system, and can realize field precision detection of the tracing device. Optionally, only one end 101 of the base 10 is provided with the positioning mechanism 30, or both ends 101 of the base 10 are provided with the positioning mechanisms 30.

Optionally, the tracing device according to the embodiments of this invention further includes a signal receiver 40 connected to the base 10. The signal receiver 40 is used for receiving a starting signal, and the tracing elements 21 transmit spatial position information according to the starting signal. The number of the signal receiver 40 can be only one. The only one signal receiver 40 is used to synchronously control the tracing elements 21 of the positioning assemblies 20. Of course, the number of the signal receiver 40 can be more than one. For instance, in some optional embodiments, the number of the signal receivers 40 is identical with that of the positioning assemblies 20, the signal receivers 40 are in one-to-one correspondence with the positioning assemblies 20, and each signal receiver 40 correspondingly controls each tracing element 21 of one positioning assembly 20.

Furthermore, this embodiment of the invention provides a positioning system. The positioning system includes an host computer, an optical position finder, a surgical robot, a calibration assembly, and the tracing device mentioned in the above embodiments. The tracing device is installed at an operation end of a mechanical arm of the surgical robot, and the calibration assembly is in a preset positional relationship with the tracing elements 21 on the tracing device in the image registration process. The host computer, through an acquired wound image including marker information of the calibration assembly and the spatial positional information of the tracing elements 21 of the tracing device acquired by the optical position finder, converts a wound image coordinate system into coordinate system referring to the tracing elements 21 or into a coordinate system referring to the optical position finder, so as to complete image registration. According to a panned path on the wound image, the host computer controls the mechanical arm to reach a target spatial position corresponding to the planned path.

Optionally, the positioning system further includes a patient tracer. The optical position finder acquires spatial positional information of the patient tracer at a certain frequency. The host computer corrects a path of the surgical robot according to the acquired spatial positional information of the patient tracer.

The positioning system of this invention includes the tracing device mentioned in the above embodiments. The tracing device can be recognized by the optical position finder within a wider range through the plurality of positioning assemblies 20 arranged in the circumferential direction X of the base 10. Meanwhile, in the tracing elements 21 of a same positioning assembly 20, the normal angle between any two of the tracing elements 21 is smaller than or equal to 20°, so that the tracing device can be more easily recognized by the optical position finder in the rotation process of the mechanical arm, and the situation that the optical position finder loses the position of the tracing device when the mechanical arm rotates is avoided The positioning precision is improved, the surgical robot can better meet surgical requirements, and thus, the positioning system is easy to use and popularize.

The invention has been described above with reference to preferred embodiments. Various improvements of the preferred embodiments and equivalent substitutes of parts in these preferred embodiments can be made without going beyond the scope of the invention. Particularly, the technical characteristics involved in these embodiments can be arbitrarily combined without structural conflicts. The invention is not limited to these specific ones disclosed above, but includes all technical solutions within the scope of the claims.

## Claims

1. A tracing device for indicating a spatial position of a mechanical arm of a surgical robot, comprising:
a base, which has two opposite ends and an outer circumferential surface located between the two ends, one end is connected to an operation end of the mechanical arm, and a surgical instrument is installed at the other end;
a positioning assembly, which is connected to the base and used for indicating a spatial position of the base, wherein the positioning assembly includes at least three tracing elements which are arranged on the outer circumferential surface of the base in a non-collinear manner;
wherein there is a plurality of positioning assemblies, and the plurality of positioning assemblies are distributed in a circumferential direction the base, and a normal angle between any two of the tracing elements of each said positioning assembly is smaller than or equal to 20°.

2. The tracing device according to Claim 1, wherein the plurality of positioning assemblies are arranged in a full circumferential direction of the base; and in every two adjacent said positioning assemblies, a normal angle between any one of the tracing elements of one positioning assembly and any one of the tracing elements of the other positioning assembly is smaller than or equal to 90°.

3. The tracing device according to Claim 1, wherein normal directions of the tracing elements of each said positioning assembly are consistent.

4. The tracing device according to Claim 3, wherein five said positioning assemblies are arranged in the full circumferential direction of the base;
in every two adjacent said positioning assemblies, the normal angle between any one of the tracing elements of one positioning assembly and any one of the tracing elements of the other positioning assembly is 72°; and/or, the angle between a vertical line from any point on an outer side of the base to the outer circumferential surface and the normal direction of each tracing elements of at least one said positioning assembly is smaller than or equal to 36°.

5. The tracing device according to Claim 1, wherein
every two adjacent said positioning assemblies are spaced from each other; or, every two adjacent said positioning assemblies are staggered with each other.

6. The tracing device according to Claim 1, wherein the at least three tracing elements of the positioning assembly are configured in a preset distribution pattern, and the distribution patterns of the tracing elements of any two said positioning assemblies are different, so that an optical position finder can distinguish the positioning assemblies according to the distribution patterns.

7. The tracing device according to Claim 1, wherein at least one end of the base is provided with a positioning assembly, which includes a positioning hole and/or positioning protrusion located at the end.

8. The tracing device according to anyone of Claims 1-7, wherein the base is of a columnar structure.

9. The tracing device according to anyone of Claims 1-7, wherein the base is provided with a plurality of installation parts, which include installation planes and/or installation holes, and each said installation part is provided with at least one of the tracing elements.

10. The tracing device according to Claim 9, wherein the base is further provided with recessed surfaces in one-to-one correspondence with the installation parts.

11. The tracing device according to anyone of Claims 1-7, wherein the base includes a first column and a second column in the axial direction of the base, the first column and the second column are arranged coaxially, an outer diameter of the first column is greater than that of the second column, and a connecting groove is formed in one end, away from the second columnar, of the first column and extends in the axial direction.

12. The tracing device according to anyone of Claims 1-7, wherein the length of a line segment formed by connecting distribution points of any two tracing elements of each said positioning assembly is greater than 40mm; and/or
an absolute value of a length difference between any two of multiple line segments formed by connecting distribution positions of any two tracing elements of each said positioning assembly is greater than 3.5mm.

13. The tracing device according to anyone of Claims 1-7, wherein the tracing device further includes a signal receiver, which is connected to the base and used for receiving a starting signal, and the tracing elements transmit spatial positional information according to the starting signal.

14. The tracing device according to anyone of Claims 1-7, wherein the tracing elements are infrared emitters or reflection balls.

15. A positioning system, including an host computer, an optical position finder, a surgical robot, and a calibration assembly, and further including the tracing device according to anyone of Claims 1-14, wherein the tracing device is installed at an operation end of a mechanical arm of the surgical robot, and the calibration assembly is in a preset positional relationship with the tracing elements on the tracing device in an image registration process;
the host computer, through an acquired wound image including marker information of the calibration assembly and the spatial positional information of the tracing elements of the tracing device acquired by the optical position finder, converts a wound image coordinate system into a coordinate system referring to the tracing elements or into a coordinate system referring to the optical position finder, so as to complete image registration; and the host computer controls the mechanical arm of the surgical robot to reach a target spatial position corresponding to a planned path according to the panned path on the wound image.

16. The positioning system according to Claim 15, wherein the positioning system further includes a patient tracer, the optical position finder acquires spatial positional information of the patient tracer at a certain frequency, and the host computer corrects the path of the surgical robot according to the acquired spatial positional information of the patient tracer.
